(19) Europäisches Patentamt
European Patent Office
Office européen des brevets

(11) Veröffentlichungsnummer: **0 031 058**
**B1**

(12) # EUROPÄISCHE PATENTSCHRIFT

(45) Veröffentlichungstag der Patentschrift: **04.04.84**

(21) Anmeldenummer: **80107588.8**

(22) Anmeldetag: **04.12.80**

(51) Int. Cl.³: **C 07 D 211/76,** C 07 D 207/38, C 07 D 207/263, C 07 D 209/46, C 07 D 217/24, C 07 D 223/10, A 61 K 31/445, A 61 K 31/395, A 61 K 31/40, A 61 K 31/47

(54) **Sulfonylharnstoffe, Verfahren zu ihrer Herstellung und pharmazeutische Präparate enthaltend diese Verbindungen.**

(30) Priorität: **19.12.79 DE 2951135**

(43) Veröffentlichungstag der Anmeldung: **01.07.81 Patentblatt 81/26**

(45) Bekanntmachung des Hinweises auf die Patenterteilung: **04.04.84 Patentblatt 84/14**

(84) Benannte Vertragsstaaten: **AT BE CH DE FR GB IT LI LU NL SE**

(56) Entgegenhaltungen:
**EP - A - 0 006 587**
**DE - A - 1 445 774**
**DE - A - 1 670 660**
**DE - A - 1 670 700**
**DE - A - 2 103 118**
**DE - A - 2 621 958**
**FR - A - 2 085 759**

**KLinik der Inneren Sekretion A. Labhart, 3. Auflage, Springer Verlag (1978), S. 740-742**
**W. Aumüller, R. Heerdt, in Handbuch der experimentellenPharmakologie, Band XX IX, S. 36, Springer Verlag Berlin, Heidelberg, New York, 1971**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Weyer, Rudi, Dr., Johann-Strauss-Strasse 43, D-6233 Kelkheim (Taunus) (DE)**
Erfinder: **Hitzel, Volker, Dr., Kantstrasse 1b, D-6238 Hofheim am Taunus (DE)**
Erfinder: **Geisen, Karl, Dr., Jahnstrasse 43, D-6000 Frankfurt am Main (DE)**
Erfinder: **Regitz, Günter, Dr., Dachbergstrasse 68, D-6232 Bad Soden am Taunus (DE)**

EP 0 031 058 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Sulfonylharnstoffe, Verfahren zu ihrer Herstellung und pharmazeutische Präparate enthaltend diese Verbindungen

Die Erfindung betrifft Sulfonylharnstoffe der Formel

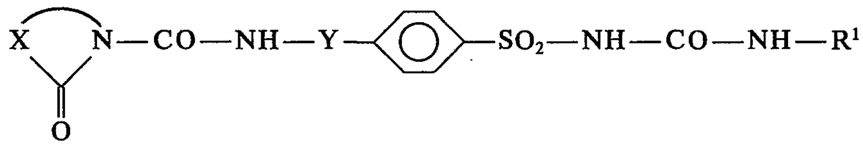

die als Substanz oder in Form ihrer physiologisch verträglichen Salze blutzuckersenkende Eigenschaften besitzen und sich durch starke Senkung des Blutzuckerspiegels auszeichnen und daher als Arzneimittel verwendet werden können.

In der Formel bedeuten:

X a) einen Alkylenrest mit 3 bis 6 C-Atomen, der gegebenenfalls mit bis zu 3 Alkylgruppen mit je 1 bis 4 C-Atomen oder mit einem Phenylrest substituiert ist,

b) einen Alkenylrest mit 3 bis 6 C-Atomen, der gegebenenfalls mit bis zu 3 Alkylgruppen mit je 1 bis 4 C-Atomen oder mit einem Phenylrest substituiert ist, oder

die Gruppe

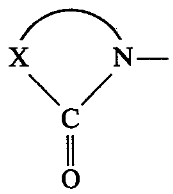

ist ein bicyclisches System der Formel

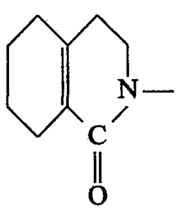

oder

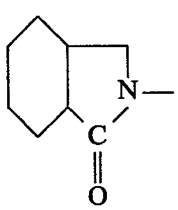

Y Alkylen mit 2–3 C-Atomen,

$R^1$ Alkyl mit 4 bis 6 C-Atomen, Cycloalkyl, Alkylcycloalkyl, Dialkylcycloalkyl, Cycloalkylalkyl, Cycloalkenyl, Alkylcycloalkenyl mit jeweils 4 bis 9 C-Atomen, Methylcyclopentylmethyl, Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl.

In der allgemeinen Formel bedeutet X vorzugsweise einen mit 1 bis 2 $C_1-C_2$-Alkylgruppen substituierten Alkylen oder Alkenylenrest mit 3 bis 4 Kohlenstoffatomen, und Y vorzugsweise $-CH_2-CH_2-$,

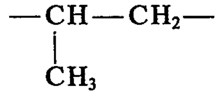

wobei die $-CH_2-CH_2$-Gruppe besonders bevorzugt ist. $R^1$ ist vorzugsweise Methylcyclopentyl,

Cyclopentylmethyl, Cyclohexyl, 4-Methyl-, 4-Äthyl-, 4-Isopropyl-cyclohexyl. Als bicyclische Reste kommen beispielsweise in Frage: Bicyclo[2.2.1]heptyl, Bicyclo[2.2.1]heptylmethyl sowie die entsprechenden ungesättigten Reste und der Bicyclo[2.2.2]octylrest.

Die Erfindung betrifft ferner Verfahren zur Herstellung dieser Sulfonylharnstoffe und pharmazeutische Präparate, die diese enthalten oder aus ihnen bestehen.

Die Verfahren zur Herstellung sind dadurch gekennzeichnet, daß man

a) mit der Gruppe

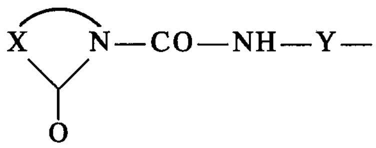

in 4-Stellung substituierte Benzolsulfonyl-isocyanate, -carbaminsäureester, -thiolcarbaminsäureester, -harnstoffe, -semicarbazide oder -semicarbazone mit einem Amin $R^1—NH_2$ oder dessen Salzen umsetzt oder Sulfonamide der Formel

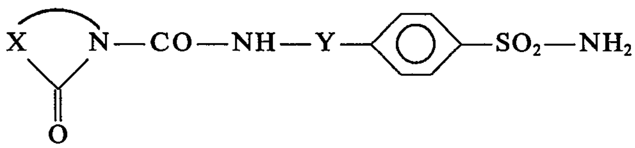

oder deren Salze mit $R^1$-substituierten Isocyanaten, Carbaminsäureestern, Thiolcarbaminsäureestern, Carbaminsäurehalogeniden oder Harnstoffen umsetzt,

b) mit der Gruppe

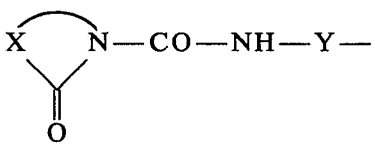

substituierte Benzolsulfonyl-isoharnstoffäther, -isothioharnstoffäther, -parabansäuren oder -halogenameisensäureamidine oder mit der Gruppe

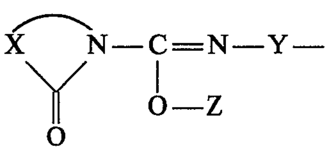

worin Z Alkyl mit 1 bis 2 Kohlenstoffatomen bedeutet, substituierte Benzolsulfonylharnstoffe spaltet,

c) in

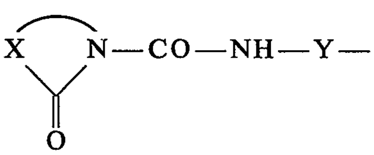

substituierten Benzolsulfonylthioharnstoffen das Schwefelatom durch Sauerstoff ersetzt,

d) entsprechende Benzolsulfinyl- oder -sulfenyl-harnstoffe oxydiert,

e) in Benzolsulfonylharnstoffe der Formel

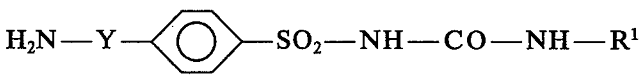

den Rest

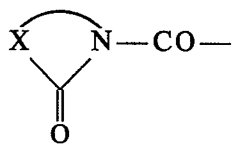

einführt,

f) entsprechend substituierte Benzolsulfonylhalogenide mit $R^1$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit $N-R^1-N'$-hydroxy-harnstoff umsetzt und die Reaktionsprodukte gegebenenfalls zur Salzbildung mit alkalischen Mitteln behandelt.

Die erwähnten Benzolsulfonyl-carbaminsäureester bzw. -thiolcarbaminsäureester können in der Alkoholkomponente einen Alkylrest oder einen Arylrest oder auch einen heterocyclischen Rest aufweisen. Da dieser Rest bei der Reaktion abgespalten wird, hat seine chemische Konstitution keinen Einfluß auf den Charakter des Endproduktes und kann deshalb in weiten Grenzen variiert werden. Das gleiche gilt für die $N-R^1$-substituierten Carbaminsäureester bzw. die entsprechenden Thiolcarbaminsäureester.

Als Carbaminsäurehalogenide eignen sich in erster Linie die Chloride.

Die als Ausgangsstoffe des Verfahrens in Frage kommenden Benzolsulfonylharnstoffe können an der der Sulfonylgruppe abgewandten Seite des Harnstoffmoleküls unsubstituiert oder ein- oder insbesondere zweifach substituiert sein. Da diese Substituenten bei der Reaktion mit Aminen abgespalten werden, kann ihr Charakter in weiten Grenzen variiert werden. Neben alkyl-, aryl-, acyl- oder heterocyclisch substituierten Benzolsulfonylharnstoffen kann man auch Benzolsulfonylcarbamoylimidazole und ähnliche Verbindungen oder Bisbenzolsulfonylharnstoffe, die an einem der Stickstoffatome noch einen weiteren Substituenten, z. B. Methyl, tragen können, verwenden. Man kann beispielsweise derartige Bis-(benzolsulfonyl)-harnstoffe oder auch N-Benzolsulfonyl-N'-acylharnstoffe mit $R^1$-substituierten Aminen behandeln und die erhaltenen Salze auf erhöhte Temperaturen, insbesondere solche oberhalb 100° C, erhitzen.

Weiterhin ist es möglich, von $R^1$-substituierten Harnstoffen auszugehen oder von solchen $R^1$-substituierten Harnstoffen, die am freien Stickstoffatom noch ein- oder insbesondere zweifach substituiert sind und diese mit

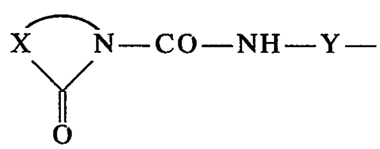

in 4-Stellung substituierten Benzolsulfonamiden umzusetzen. Als solche Ausgangsstoffe kommen beispielsweise in Frage N-Cyclohexyl-harnstoff, die entsprechenden N'-Acetyl, N'-Nitro, N'-Cyclohexyl, N',N'-Diphenyl- (wobei die beiden Phenylreste auch substituiert sowie direkt oder auch über ein Brückenglied wie $-CH_2-$, $-NH-$, $-O-$ oder $-S-$ miteinander verbunden sein können), N'-Methyl-N'-phenyl-, N,N-Dicyclohexylharnstoffe sowie Cyclohexyl-carbamoylimidazole, -pyrazole oder -triazole sowie solche der genannten Verbindungen, die anstelle des Cyclohexyls einen anderen im Bereich der Definition für $R^1$ liegenden Substituenten tragen.

Die Spaltung der als Ausgangsstoffe in Verfahren b) genannten Benzolsulfonylparabansäuren, -isoharnstoffäther, -isothioharnstoffäther oder -halogenameisensäureamidine sowie der genannten Benzolsulfonylharnstoffe erfolgt zweckmäßig durch alkalische Hydrolyse. Isoharnstoffäther können auch in einem sauren Medium mit gutem Erfolg gespalten werden.

Der Ersatz des Schwefelatoms in der Thioharnstoffgruppierung von entsprechend substituierten Benzolsulfonylthioharnstoffen durch ein Sauerstoffatom kann in bekannter Weise zum Beispiel mit Hilfe von Oxyden oder Salzen von Schwermetallen oder auch durch Anwendung von Oxydationsmitteln, wie Wasserstoffperoxid, Natriumperoxid, salpetriger Säure oder Permanganaten ausgeführt werden. Die Thioharnstoffe können auch entschwefelt werden durch Behandlung mit Phosgen oder Phosphorpentachlorid. Als Zwischenstufe erhaltene Chlorameisensäureamidine bzw. Carbodiimide können durch geeignete Maßnahmen wie Verseifen oder Anlagerung von Wasser in die Benzolsulfonylharnstoffe überführt werden.

Die Oxydation von Benzolsulfinyl- bzw. Benzolsulfenylharnstoffen erfolgt nach an sich bekannter Methode, vorzugsweise mit Oxydationsmitteln wie Permanganat oder Wasserstoffperoxid.

Die Acylierung der Sulfonylharnstoffe gemäß Verfahren e) kann mit reaktiven Derivaten der Säure

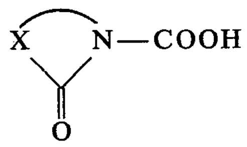

wie beispielsweise Halogeniden, erfolgen.

Als Sulfonyl- bzw. Sulfinylhalogenide gemäß Verfahren f) eignen sich insbesondere die Chloride. Als saures Kondensationsmittel kann man beispielsweise Thionylchlorid oder Polyphosphorsäure einsetzen.

Die Herstellung der physiologisch verträglichen Salze erfolgt nach an sich bekannten Methoden. Zur Salzbildung sind insbesondere geeignet Alkali- und Erdalkalihydroxyde, -carbonate oder -bicarbonate sowie physiologisch verträgliche organische Basen.

Die Ausführungsformen des Verfahrens gemäß der Erfindung können im allgemeinen hinsichtlich der Reaktionsbedingungen weitgehend variiert und den jeweiligen Verhältnissen angepaßt werden. Beispielsweise können die Umsetzungen in Abwesenheit oder Anwesenheit von Lösungsmitteln, bei Raumtemperatur oder bei erhöhter Temperatur durchgeführt werden.

Je nach dem Charakter der Ausgangsstoffe kann das eine oder andere der beschriebenen Verfahren in einzelnen Fällen einen gewünschten individuellen Benzolsulfonylharnstoff nur in geringen Ausbeuten liefern oder zu dessen Synthese nicht geeignet sein. In solchen verhältnismäßig selten auftretenden Fällen macht es dem Fachmann keine Schwierigkeiten, das gewünschte Produkt auf einem anderen der beschriebenen Verfahrenswege zu synthetisieren.

Die erhaltenen Verbindungen können durch Umfällen und/oder Umkristallisieren gereinigt werden. Die Reinigung kann auch erfolgen, indem man die Substanz aus einem kristallinen (Alkali)-Salz in einem geeigneten Lösungsmittel in Freiheit setzt.

Die erfindungsgemäßen Verbindungen zeichnen sich durch wertvolle pharmakologische Eigenschaften, insbesondere blutzuckersenkende, aus. Sie eignen sich daher als Arzneimittel, insbesondere als Antidiabetika.

Die blutzuckersenkende Wirkung der erfindungsgemäßen Benzolsulfonylharnstoffe kann dadurch festgestellt werden, daß man sie als freie Verbindungen oder in Form der Natriumsalze an normal ernährte Kaninchen verfüttert und den Blutzuckerwert nach der bekannten Methode von Hagedorn-Jensen oder mit einem Autoanalyzer über eine längere Zeitdauer ermittelt.

Die routinemäßige Bestimmung der blutzuckersenkenden Wirkung kann mit Dosierungen von z. B. 10,2 oder 0,4 mg Wirksubstanz pro kg Versuchstier nach bekannten Methoden erfolgen.

Die folgenden Verbindungen I bis III wurden in Dosierungen von 2 mg/kg Kaninchen oral verabreicht und die Blutzuckerwerte wurden mit einem Autoanalyzer über eine längere Zeitdauer ermittelt. Die hierbei gemessene Blutzuckersenkung ist in der nachfolgenden Tabelle in % nach ... Stunden angegeben.

I. N-(4-<2-(3-Äthyl-4-methyl-2-oxo-3-pyrrolin-1-carboxamido)-äthyl>-benzolsulfonyl)-N'-cyclohexylharnstoff

II. N-(4-<2-(3-Äthyl-4-methyl-2-oxo-3-pyrrolin-1-carboxamido)-äthyl>-benzolsulfonyl)-N'-4-methylcyclohexylharnstoff

III. N-(4-<2-(3,4-Dimethyl-2-oxo-3-pyrrolin-1-carboxamido)-äthyl>-benzolsulfonyl)-N'-4-methylcyclohexyl-harnstoff

Tabelle

| Verbindung | Blutzuckersenkung am Kaninchen nach Verabreichung von 2 mg/kg p.o. in % nach | | | | | | | |
|---|---|---|---|---|---|---|---|---|
| | 1 | 3 | 6 | 24 | 48 | 72 | 92 | 120 Std. |
| I | 34 | 45 | 37 | 44 | 32 | 8 | | |
| II | 24 | 35 | 36 | 33 | 34 | 8 | 0 | |
| III | 32 | 34 | 38 | 41 | 68 | 41 | 25 | 17 |

Die erfindungsgemäßen Acylureidoalkylbenzolsulfonylharnstoffe zeichnen sich durch eine starke blutzuckersenkende Wirkung aus. Außerdem sind die Verbindungen gut verträglich.

Die Eigenschaften der Verbindungen erlauben es, in der Therapie des Diabetes mellitus mit so geringen Dosen auszukommen, daß das Präparat nur die verminderte Ansprechbarkeit des Pankreas auf einen erhöhten Blutzuckerspiegel wieder normalisiert.

Benzolsulfonylharnstoffe, die eine Acylaminoalkylgruppe tragen, werden z. B. beschrieben in DE-OS 2 621 958, DE-OS 2 103 118, DE-OS 1 445 774 und DE-OS 1 670 660. Die erfindungsgemäßen Verbindungen unterscheiden sich von den beschriebenen Verbindungen dadurch, daß die Aminoalkylgruppe ersetzt wurde durch die Ureidoalkylgruppe. Ferner wurden andere Acylreste eingeführt. Diese Änderungen führten zu Verbindungen, die bei niedrigerer Dosierung eine starke und lang andauernde Blutzuckersenkung bewirken.

Benzolsulfonylharnstoffe mit Ureidoalkylrest sind auch schon mehrfach beschrieben worden (DE-OS 1 443 911, DE-AS 1 670 700, DE-PS 1 618 389, DE-PS 2 238 870). Die aus der DE-AS 1 670 700 bekannten Ureidoalkylbenzolsulfonylharnstoffe sind niemals mit einem Acylrest substituiert. Bei einer Dosierung von 10 mg/kg Kaninchen bewirken sie eine Blutzuckersenkung, die nicht so lange andauert und so stark ausgeprägt ist wie diejenige, die nach Verabreichung von 2 mg/kg Kaninchen der erfindungsgemäßen Verbindungen beobachtet wurde.

Wenn auch in Klinik der inneren Sekretion (Springer Verlag, Berlin, Heidelberg, New York 1978), Seiten 740—742, ausgeführt wird, daß die Anwesenheit eines Benzolringes, einer Sulfonylgruppe und eines Harnstoffrestes wesentlich für die Wirkungsweise ist, und nur die Seitengruppe die Wirkungsdauer und Wirksamkeit beeinflussen, so war es überraschend, daß die Acylierung der Ureidoalkylbenzolsulfonylharnstoffe zu so stark wirksamen Verbindungen führt, da eher ein starker Abfall der Wirksamkeit zu erwarten war, denn die Acylierung von Sulfonylharnstoffen zu Sulfonylacyl-harnstoffen führte zu Verbindungen mit weniger ausgeprägter blutzuckersenkender Wirkung (W. Aumüller, R. Heerdt, in Handbuch der experimentellen Pharmakologie, Band XXIX, S. 36, Springer Verlag Berlin, Heidelberg, New York, 1971). Es war nicht zu erwarten, daß die erfindungsgemäßen Verbindungen sich durch die obenerwähnten günstigen Eigenschaften auszeichnen.

Die beschriebenen Sulfonylharnstoffe sollen vorzugsweise zur Herstellung von oral verabreichbaren Präparaten zur Behandlung des Diabetes mellitus dienen. Sie können als solche oder in Form ihrer Salze bzw. in Gegenwart von Stoffen, die zu einer Salzbildung führen, appliziert werden. Zur Salzbildung können beispielsweise alkalische Mittel wie Alkali- oder Erdalkalihydroxyde, -carbonate oder -bicarbonate herangezogen werden. Die Präparate können neben dem Sulfonylharnstoff bzw. dessen Salz auch noch andere Wirkstoffe enthalten.

Als medizinische Präparate kommen vorzugsweise Tabletten in Betracht, die neben den Sulfonylharnstoffen oder deren Salzen die üblichen Träger- und Hilfsstoffe wie Talkum, Stärke, Milchzucker oder Magnesiumstearat enthalten. Dabei kann es zweckmäßig sein, den oder die Wirkstoffe in gemahlener oder fein gefällter Form oder als Gemisch dieser Formen einzusetzen. Ein Präparat, das die erfindungsgemäßen Benzolsulfonylharnstoffe als Wirkstoff enthält, z. B. eine Tablette oder ein Pulver mit oder ohne Zusätze, ist zweckmäßig in eine geeignet dosierte Form gebracht. Als Dosis ist dabei eine solche zu wählen, die der Wirksamkeit des verwendeten Benzolsulfonylharnstoffs und dem gewünschten Effekt angepaßt ist. Zweckmäßig beträgt die Dosierung je Einheit etwa 0,5 bis 50 mg, vorzugsweise 1 bis 20 mg, jedoch können auch darüber oder darunter liegende Dosierungseinheiten verwendet werden, die gegebenenfalls vor Applikation zu teilen bzw. zu vervielfachen sind.

Die nachfolgenden Beispiele zeigen einige der zahlreichen Verfahrensvarianten, die zur Synthese der erfindungsgemäßen Sulfonylharnstoffe verwendet werden können.

### Beispiel 1

N-(4-(2-(2-Oxo-pyrrolidin-1-carboxamido)-äthyl)-benzolsulfonyl)-N'-cyclohexyl-harnstoff

47 g 2-Oxo-pyrrolidin-1-(N-2-phenyl-äthyl)-carboxamid (Schmp. 88—90°, hergestellt aus Pyrrolidon und 2-Phenyl-äthyl-isocyanat) werden unter Kühlung und Rühren in 95 g Chlorsulfonsäure eingetragen. Anschließend erhitzt man 1 Std. auf 50°C, gießt nach Abkühlen auf Eis, trennt das Sulfochlorid ab und behandelt es mit konz. Ammoniak. Das Sulfonamid wird abgesaugt und aus Butylacetat-Methylglykol umkristallisiert. Schmp. 178—180°.

5 g 4-(2-(2-Oxo-pyrrolidin-1-carboxamido)-äthyl)-benzolsulfonamid werden in 100 ml Aceton mit 0,65 g NaOH und Wasser in Lösung gebracht. Dazu tropft man unter Rühren und Eiskühlung 2,3 g Cyclohexylisocyanat und rührt 2 Std. bei Raumtemperatur nach. Anschließend destilliert man das Aceton größtenteils unter vermindertem Druck ab, säuert die wäßrige Lösung an und fällt das Produkt aus verdünntem Ammoniak um. Der erhaltene N-(4-(2-(2-Oxo-pyrrolidin-1-carboxamido)-äthyl)-benzolsulfonyl)-N'-cyclohexyl-harnstoff wird aus verd. Äthanol umkristallisiert und schmilzt bei 189—190°.

In analoger Weise erhält man den

N-(4-(2-(2-Oxo-pyrrolidin-1-carboxamido)-äthyl)-benzolsulfonyl)-N'-4-methyl-cyclohexyl-harnstoff
vom Schmp. 179—181° (aus verd. Äthanol).

Beispiel 2

N-(4-(2-(2-Oxo-piperidin-1-carboxamido)-äthyl)-benzolsulfonyl)-N'-cyclohexyl-harnstoff

50,4 g Piperidon-(2) und 73 g β-Phenyläthylisocyanat werden 2 Std. auf 150°C im Ölbad erhitzt. Die klare Schmelze wird abgekühlt, mit Petroläther behandelt, das Kristallisat abgesaugt und das so erhaltene 2-Oxo-piperidin-1-(N-2-phenyläthyl)-carboxamid aus Diisopropyläther umkristallisiert, Schmp. 66–67°.

49,2 g 2-Oxo-piperidin-1-(N-2-phenyläthyl)-carboxamid werden bei 30° portionsweise in 140 g Chlorsulfonsäure eingetragen und 1 Std. bei 40° gerührt.

Die klare zähe Lösung wird auf Eis getropft, das Sulfonsäurechlorid abgesaugt (Schmp. 134–136°), in 750 ml conc. Ammoniak eingetragen und 30 Minuten auf dem Dampfbad erhitzt.

Das 4-(2-(2-Oxo-piperidin-1-carboxamido)-äthyl)-benzolsulfonamid wird abgesaugt, aus Isopropanol umkristallisiert, Schmp. 173–174°.

3,3 g Sulfonamid, 80 ml Aceton und 2,8 g $K_2CO_3$ werden 6 Std. unter Rühren und Rückfluß zum Sieden erhitzt. Anschließend tropft man 1,3 g Cyclohexylisocyanat zu und rührt 6 Std. bei Siedetemperatur nach.

Nach Stehen über Nacht saugt man ab und behandelt das erhaltene Kristallisat mit verd. Salzsäure und saugt wieder ab.

Der in guter Ausbeute erhaltene N-(4-(2-(2-Oxo-piperidin-1-carboxamido)-äthyl)-benzolsulfonyl)-N'-cyclohexyl-harnstoff wird aus sehr verd. wäßr. Ammoniak/verd. Salzsäure umgefällt und aus wäßr. Methanol umkristallisiert, Schmp. 197–199°.

In analoger Weise erhält man den

N-(4-(2-(2-Oxo-piperidin-1-carboxamido)-äthyl)-benzolsulfonyl)-N'-4-methyl-cyclohexyl-harnstoff
vom Schmp. 180–182° (aus wäßr. Äthanol),
N-(4-(2-(2-Oxo-piperidin-1-carboxamido)-äthyl)-benzolsulfonyl)-N'-butyl-harnstoff
Schmp. 156–158° (aus Aceton/Wasser),
N-(4-(2-(2-Oxo-piperidin-1-carboxamido)-äthyl)-benzolsulfonyl)-N'-cyclopentyl-harnstoff
Schmp. 167–169° (aus wäßr. Methanol),
N-(4-(2-(2-Oxo-piperidin-1-carboxamido)-äthyl)-benzolsulfonyl)-N'-isobutyl-harnstoff
Schmp. 179–181° (aus Aceton/Wasser),
N-(4-(2-(2-Oxo-piperidin-1-carboxamido)-äthyl)-benzolsulfonyl)-N'-cyclooctyl-harnstoff
Schmp. 170–172° (aus Aceton/Wasser).

Beispiel 3

N-(4-(2-(2-Oxo-piperidin-1-carboxamido)-äthyl)-benzolsulfonyl)-N'-3,4-dimethylcyclohexyl-harnstoff

2,3 g N-(4-(2-(2-Oxo-piperidin-1-carboxamido)-äthyl)-benzolsulfonyl)-carbaminsäure-methylester (Schmp. 167–169°, dargestellt aus dem entsprechenden Sulfonamid mit Chlorameisensäuremethylester und Aceton in Gegenwart von Kaliumcarbonat) 50 ml Dioxan und 0,8 g 3,4-Dimethyl-cyclohexylamin werden 1 Std. unter Rückfluß zum Sieden erhitzt. Das Lösungsmittel wird unter vermindertem Druck abdestilliert, der Rückstand aus sehr verd. Ammoniak/verd. Salzsäure umgefällt und der in guter Ausbeute erhaltene N-(4-(2-(2-Oxo-piperidin-1-carboxamido)-äthyl)-benzolsulfonyl)-N'-3,4-dimethylcyclohexyl-harnstoff aus Aceton/Wasser umkristallisiert, Schmp. 139–141°.

In analoger Weise erhält man den

N-(4-(2-(2-Oxo-piperidin-1-carboxamido)-äthyl)-benzolsulfonyl)-N'-cyclopentylmethyl-harnstoff
Schmp. 172–174° (aus Aceton/Wasser),
N-(4-(2-(2-Oxo-piperidin-1-carboxamido)-äthyl)-benzolsulfonyl)-N'-nortricyclyl-harnstoff
Schmp. 179–181° (aus Aceton/Wasser),
N-(4-(2-(2-Oxo-piperidin-1-carboxamido)-äthyl)-benzolsulfonyl)-N'-bicyclo-[2.2.1]-hept-5-en-2-yl-methylharnstoff
Schmp. 184–186° (aus Aceton/Wasser),
N-(4-(2-(2-Oxo-piperidin-1-carboxamido)-äthyl)-benzolsulfonyl)-N'-hexyl-

harnstoff
Schmp. 142–144° (aus Aceton/Wasser),
N-(4-(2-(2-Oxo-piperidin-1-carboxamido)-äthyl)-benzolsulfonyl)-N'-3-methyl-cyclopentyl-harnstoff
Schmp. 166–168° (aus Aceton/Wasser),
N-(4-(2-(2-Oxo-piperidin-1-carboxamido)-äthyl)-benzolsulfonyl)-N'-cyclohex-3-enyl-methyl-harnstoff (x 1/2 $H_2O$)
Schmp. 136–138° (aus Aceton/Wasser),
N-(4-(2-(2-Oxo-piperidin-1-carboxamido)-äthyl)-benzolsulfonyl)-N'-4-isopropyl-cyclohexyl-harnstoff
Schmp. 158–160° (aus Aceton/Wasser).

## Beispiel 4

### N-(4-(2-(2-Oxo-hexamethylenimin-1-carboxamido)-äthyl)-benzolsulfonyl)-N'-cyclohexyl-harnstoff

28,2 g Caprolactam und 37 g 2-Phenyl-äthylisocyanat werden 2 Std. auf 150° im Ölbad erhitzt, das Reaktionsgut auf Wasser gegossen, das 2-Oxo-hexamethylenimin-1-(N-2-phenyläthyl)-carboxamid abgesaugt, getrocknet und aus Petroläther umkristallisiert, Schmp. 65–67°.

43 g 2-Oxo-hexamethylenimin-1-(N-2-phenyläthyl)-carboxamid werden bei ca. 30° portionsweise in 80 ml Chlorsulfonsäure eingetragen. Man rührt 1 Std. bei 50° nach, kühlt ab und tropft das Reaktionsgemisch auf Eiswasser. Das schmierig ausgefallene Sulfonsäurechlorid wird mit 300 ml konz. Ammoniak versetzt und 30 Min. auf dem Dampfbad erhitzt.

Das erhaltene Sulfonamid wird abgesaugt und getrocknet. Schmp. 176–178° (aus verdünntem Methanol).

3,4 g 4-(2-(2-Oxo-hexamethylenimin-1-carboxamido)-äthyl)-benzolsulfonamid, 150 ml Aceton und 2,8 g $K_2CO_3$ werden 6 Std. unter Rühren und Rückfluß zum Sieden erhitzt. Anschließend tropft man 1,3 g Cyclohexylisocyanat zu und rührt 6 Std. bei Siedetemperatur nach.

Das K-Salz des Harnstoffs wird abgesaugt, in Wasser gelöst, die Lösung filtriert und das Filtrat mit verd. Salzsäure angesäuert. Der in guter Ausbeute erhaltene Sulfonylharnstoff wird abgesaugt, aus sehr verd. wäßr. Ammoniak/verd. Salzsäure umgefällt und aus Äthanol umkristallisiert. Schmp. 179–181°.

In analoger Weise erhält man den

N-(4-(2-(2-Oxo-hexamethylenimin-1-carboxamido)-äthyl)-benzolsulfonyl)-N'-4-methyl-cyclohexyl-harnstoff
vom Schmp. 181–182° (aus Aceton/Wasser),
N-(4-(2-(2-Oxo-hexamethylenimin-1-carboxamido)-äthyl)-benzolsulfonyl)-N'-butyl-harnstoff
vom Schmp. 117–119° (aus Äthanol),
N-(4-(2-(2-Oxo-hexamethylenimin-1-carboxamido)-äthyl)-benzolsulfonyl)-N'-4-äthyl-cyclohexylharnstoff
vom Schmp. 162–164° (aus verd. Aceton),
N-(4-(2-(2-Oxo-hexamethylenimin-1-carboxamido)-äthyl)-benzolsulfonyl)-N'-3-methyl-cyclopentylharnstoff
vom Schmp. 161–163° (aus verd. Aceton).

## Beispiel 5

### N-(4-(2-(2-Oxo-hexamethylenimin-1-carboxamido)-äthyl)-benzolsulfonyl)-N'-cyclopentyl-harnstoff

4 g N-(4-(2-(2-Oxo-hexamethylenimin-1-carboxamido)-äthyl)-benzolsulfonyl)-carbaminsäure-methylester (Schmp. 146–148°, dargestellt aus dem Sulfonamid mit Chlorameisensäure-methylester und Kaliumcarbonat in Aceton bei Siedetemperatur) 100 ml Dioxan und 0,9 g Cyclopentylamin werden 1 Std. unter Rückfluß zum Sieden erhitzt. Das Lösungsmittel wird im Vakuum abdestilliert, der Rückstand aus sehr verd. wäßr. Ammoniak/verd. Salzsäure umgefällt. Der in guter Ausbeute erhaltene N-(4-(2-(2-Oxo-hexamethylenimin-1-carboxamido)-äthyl)-benzolsulfonyl)-N'-cyclopentyl-harnstoff wird abgesaugt und aus Aceton/Wasser umkristallisiert. Schmp. 148–150°.

In analoger Weise erhält man den

N-(4-(2-(2-Oxo-hexamethylenimin-1-carboxamido)-äthyl)-benzolsulfonyl)-N'-hexyl-harnstoff
Schmp. 142–144° (aus Aceton/Wasser),
N-(4-(2-Oxo-hexamethylenimin-1-carboxamido)-äthyl)-benzolsulfonyl)-N'-4-isopropyl-cyclohexylharnstoff
vom Schmp. 173–175° (aus verd. Aceton).

Beispiel 6

N-(4-(2-(1-Oxo-1,2,3,4,5,6,7,8-octahydro-isochinolin-2-carboxamido)-äthyl)-benzolsulfonyl)-N'-cyclohexylharnstoff

1,9 g 4-(2-(1-Oxo-1,2,3,4,5,6,7,8-octahydro-isochinolin-2-carboxamido)-äthyl)-benzolsulfonamid (Schmp. 140–142° C, hergestellt aus 1-Oxo-1,2,3,4,5,6,7,8-octahydro-isochinolin-2-(N-2-phenyläthyl)-carboxamid (Schmp. 47° C, erhalten durch Reaktion von 1-Oxo-1,2,3,4,5,6,7,8-octahydro-isochinolin und Phenyläthylisocyanat) mit Chlorsulfonsäure und Umsetzung des erhaltenen Sulfochlorids mit Ammoniak) werden in 80 ml Aceton nach Zusatz von 1,4 g gemahlener Pottasche 4 Stunden unter Rückfluß gerührt. Nach kurzem Abkühlen tropft man eine Lösung von 0,7 g Cyclohexylisocyanat in wenig Aceton zu und rührt weitere 4 Stunden unter Rückfluß nach. Die Suspension wird eingedampft, der Rückstand mit Wasser in Lösung gebracht und die Lösung mit 2 N Salzsäure angesäuert. Der Niederschlag wird abgesaugt und nach Umfällen aus verdünnter Ammoniak-Lösung mit 2 N Salzsäure aus Äthanol umkristallisiert. Der so erhaltene N-(4-(2-(1-Oxo-1,2,3,4,5,6,7,8-octahydro-isochinolin-2-carboxamido)-äthyl)-benzolsulfonyl)-N'-cyclohexylharnstoff schmilzt bei 176–177° C.
In analoger Weise erhält man den

N-(4-(2-(1-Oxo-1,2,3,4,5,6,7,8-octahydro-isochinolin-2-carboxamido)-äthyl)-benzolsulfonyl)-N'-(4-methyl-cyclohexyl)-harnstoff
vom Schmp. 180–182° C (aus Äthanol).

Beispiel 7

N-(4-(2-(1-Oxo-hexahydro-isoindolin-2-carboxamido)-äthyl)-benzolsulfonyl)-N'-cyclohexyl-harnstoff

2,74 g 4-(2-(1-Oxo-hexahydro-isoindolin-2-carboxamido)-äthyl)-benzolsulfonamid (Schmp. 145–147° C, hergestellt aus 1-Oxo-hexahydro-isoindolin-2-yl-(N-2-phenyl-äthyl)-carboxamid (Schmp. 65–68° C, erhalten durch Reaktion von 1-Oxo-hexahydro-isoindolin und Phenyläthylisocyanat) mit Chlorsulfonsäure und Umsetzung des erhaltenen Sulfochlorids mit Ammoniak) werden in 50 ml Aceton und 25 ml Dioxan nach Zusatz von 2,1 g gemahlener Pottasche 3 Stunden unter Rückfluß gerührt. Nach kurzem Abkühlen tropft man eine Lösung von 1,1 g Cyclohexylisocyanat in wenig Aceton zu und hält weitere 4 Stunden im Rückfluß. Die erkaltete Suspension wird im Vakuum eingeengt, der Rückstand in Wasser gelöst und die Lösung mit 2 N Salzsäure angesäuert. Der Niederschlag wird nach dem Absaugen aus verdünnter Ammoniak-Lösung mit 2 N Salzsäure umgefällt und aus Äthanol umkristallsiert. Der so hergestellte N-(4-(2-(1-Oxo-hexahydro-isoindolin-2-carboxamido)-äthyl)-benzolsulfonyl)-N'-cyclohexyl-harnstoff schmilzt bei 139–140° C.
In analoger Weise erhält man den

N-(4-(2-(1-Oxo-hexahydro-isoindolin-2-carboxamido)-äthyl)-benzolsulfonyl)-N'-(4-methyl-cyclohexyl)-harnstoff
vom Schmp. 153–155° C (aus Äthanol).

Beispiel 8

N-(4-(2-(4-Methyl-2-oxo-piperidin-1-carboxamido)-äthyl)-benzolsulfonyl)-N'-cyclohexyl-harnstoff

Entsprechend Beispiel 2 erhält man aus 4-Methyl-2-oxo-piperidin und 2-Phenyläthylisocyanat das 4-Methyl-2-oxo-piperidin-1-(N-2-phenyläthyl)-carboxamid vom Schmp. 63°, hieraus mit Chlorsulfonsäure das 4-(2-(4-Methyl-2-oxo-piperidin-1-carboxamido)-äthyl)-benzolsulfochlorid vom Schmp. 99–101°, hieraus mit Ammoniak das entsprechende Sulfonamid vom Schmp. 149–151°, hieraus mit Cyclohexylisocyanat den

N-(4-(2-(4-Methyl-2-oxo-piperidin-1-carboxamido)-äthyl)-benzolsulfonyl)-N'-cyclohexyl-harnstoff
vom Schmp. 177—178° (aus verd. Aceton).

In analoger Weise erhält man aus dem Sulfonamid den

N-(4-(2-(4-Methyl-2-oxo-piperidin-1-carboxamido)-äthyl)-benzolsulfonyl)-
N'-4-methylcyclohexyl-harnstoff
vom Schmp. 190—191° (aus verd. Aceton),
N-4-(2-(4-Methyl-2-oxo-piperidin-1-carboxamido)-äthyl)-benzolsulfonyl)-
N'-butyl-harnstoff
vom Schmp. 159—161° (aus verd. Aceton),
N-(4-(2-(4-Methyl-2-oxo-piperidin-1-carboxamido)-äthyl)-benzolsulfonyl)-
N'-4-äthyl-cyclohexyl-harnstoff
vom Schmp. 178—180° (aus verd. Methanol),
N-(4-(2-(4-Methyl-2-oxo-piperidin-1-carboxamido)-äthyl)-benzolsulfonyl)-
N'-4-methyl-cyclohex-3-enyl-harnstoff
vom Schmp. 173—175° (aus verd. Aceton).

## Beispiel 9

N-(4-(2-(4-Methyl-2-oxo-piperidin-1-carboxamido)-äthyl)-benzolsulfonyl)-
N'-cyclopentyl-harnstoff

Entsprechend Beispiel 3 erhält man aus dem N-(4-(2-(4-Methyl-2-oxo-piperidin-1-carboxamido)-äthyl)-benzolsulfonyl)-carbaminsäuremethylester (Schmp. 137—139°, hergestellt aus dem in Beispiel 8 genannten 4-(2-(4-Methyl-2-oxo-piperidin-1-carboxamido)-äthyl)-benzolsulfonamid durch Umsetzung mit Chlorameisensäuremethylester) den

N-(4-(2-(4-Methyl-2-oxo-piperidin-1-carboxamido)-äthyl)-benzolsulfonyl)-
N'-cyclopentyl-harnstoff
vom Schmp. 177—179° (aus verd. Methanol),
N-(4-(2-(4-Methyl-2-oxo-piperidin-1-carboxamido)-äthyl)-benzolsulfonyl)-
N'-4-isopropylcyclohexyl-harnstoff
vom Schmp. 171—173° (aus Methanol),
N-(4-(2-(4-Methyl-2-oxo-piperidin-1-carboxamido)-äthyl)-benzolsulfonyl)-
N'-cycloheptyl-harnstoff
vom Schmp. 142—143° (aus verd. Äthanol),
N-(4-(2-(4-Methyl-2-oxo-piperidin-1-carboxamido)-äthyl)-benzolsulfonyl)-
N'-3-methyl-cyclopentyl-harnstoff
vom Schmp. 145—147° (aus verd. Aceton),
N-(4-(2-(4-Methyl-2-oxo-piperidin-1-carboxamido)-äthyl)-benzolsulfonyl)-
N'-bicyclo[2.2.1]hep-5-en-2-yl-methylharnstoff
vom Schmp. 164—166° (aus verd. Aceton),
N-(4-(2-(4-Methyl-2-oxo-piperidin-1-carboxamido)-äthyl)-benzolsulfonyl)-
N'-3-methyl-cyclopentylmethyl-harnstoff
vom Schmp. 152—154° (aus verd. Aceton),
N-(4-(2-(4-Methyl-2-oxo-piperidin-1-carboxamido)-äthyl)-benzolsulfonyl)-
N'-adamantyl-harnstoff
vom Schmp. 176—178° (aus verd. Aceton).

## Beispiel 10

N-(4-(2-(3-Butyl-4-methyl-2-oxo-piperidin-1-carboxamido)-äthyl)-benzolsulfonyl)-
N'-cyclohexyl-harnstoff

3-Butyl-4-methyl-2-oxo-piperidin (Schmp. 95—98°, hergestellt analog DE-OS 1 023 464) wird analog Beispiel 2 mit Phenyläthylisocyanat zum 3-Butyl-4-methyl-2-oxo-piperidin-1-(N-2-phenyläthyl)-carboxamid, dieses als Rohprodukt mit Chlorsulfonsäure zum Sulfochlorid, das Sulfochlorid mit Ammoniak zum 4-(2-(3-Butyl-4-methyl-2-oxo-piperidin-1-carboxamido)-äthyl)-benzolsulfonamid, Schmp. 165—167° umgesetzt und hieraus mit Cyclohexylisocyanat der

N-(4-(2-(3-Butyl-4-methyl-2-oxo-piperidin-1-carboxamido)-äthyl)-benzolsulfonyl)-
N'-cyclohexyl-harnstoff

vom Schmp. 123 – 125° (aus Methanol)

erhalten.

In analoger Weise erhält man aus dem Sulfonamid den

N-(4-(2-(3-Butyl-4-methyl-2-oxo-piperidin-1-carboxamido)-äthyl)-benzolsulfonyl)-
N'-4-methyl-cyclohexyl-harnstoff
vom Schmp. 131 – 133° (aus Methanol).

Beispiel 11

N-(4-(2-(3,4-Dimethyl-2-oxo-3-pyrrolin-1-carboxamido)-äthyl)-benzolsulfonyl)-
N'-cyclohexyl-harnstoff

Entsprechend Beispiel 2 erhält man aus 3,4-Dimethyl-2-pyrrolon und 2-Phenyläthylisocyanat das 3,4-Dimethyl-2-oxo-3-pyrrolin-1-(N-2-phenyläthyl)-carboxamid vom Schmp. 132 – 134°, daraus mit Chlorsulfonsäure das Sulfochlorid vom Schmp. 189 – 190°, aus dem Sulfochlorid mit Ammoniak das Sulfonamid vom Schmp. 232 – 234°, und aus dem Sulfonamid mit Cyclohexylisocyanat den

N-(4-(2-(3,4-Dimethyl-2-oxo-3-pyrrolin-1-carboxamido)-äthyl)-benzolsulfonyl)-
N'-cyclohexylharnstoff
vom Schmp. 200 – 202° (aus Methanol-Dioxan).

In analoger Weise erhält man aus dem Sulfonamid den

N-(4-(2-(3,4-Dimethyl-2-oxo-3-pyrrolin-1-carboxamido)-äthyl)-benzolsulfonyl)-
N'-4-methyl-cyclohexylharnstoff
vom Schmp. 208 – 210° (aus verd. Methanol),
N-(4-(2-(3,4-Dimethyl-2-oxo-3-pyrrolin-1-carboxamido)-äthyl)-benzolsulfonyl)-
N'-butylharnstoff
vom Schmp. 198 – 200° (aus Methanol-Dioxan).

Beispiel 12

N-(4-(2-(4-Methyl-2-oxo-3-pyrrolin-1-carboxamido)-äthyl)-benzolsulfonyl)-
N'-cyclohexylharnstoff

Entsprechend Beispiel 2 erhält man aus 4-Methyl-2-pyrrolon und 2-Phenyläthylisocyanat das 4-Methyl-2-oxo-3-pyrrolin-1-(N-2-phenyläthyl)-carboxamid vom Schmp. 94 – 96°, daraus mit Chlorsulfonsäure das Sulfochlorid vom Schmp. 193 – 195°, aus dem Sulfochlorid das Sulfonamid vom Schmp. 196 – 198° und aus dem Sulfonamid mit Cyclohexylisocyanat den

N-(4-(2-(4-Methyl-2-oxo-3-pyrrolin-1-carboxamido)-äthyl)-benzolsulfonyl)-
N'-cyclohexylharnstoff
vom Schmp. 195 – 197° (aus verd. Methanol-Dioxan).

In analoger Weise erhält man aus dem Sulfonamid den

N-(4-(2-(4-Methyl-2-oxo-3-pyrrolin-1-carboxamido)-äthyl)-benzolsulfonyl)-
N'-4-methyl-cyclohexylharnstoff
vom Schmp. 199 – 201° (aus verd. Dioxan),
N-(4-(2-(4-Methyl-2-oxo-3-pyrrolin-1-carboxamido)-äthyl)-benzolsulfonyl)-
N'-butyl-harnstoff
vom Schmp. 189 – 191° (aus verd. Methanol-Dioxan).

Beispiel 13

N-(4-(2-(2-Oxo-piperidin-1-carboxamido)-propyl)-benzolsulfonyl)-N'-cyclohexyl-harnstoff

1,6 g 4-(2-(2-Oxo-piperidin-1-carboxamido)-propyl)-benzolsulfonamid (Schmp. 165 – 167°, hergestellt aus 4-(2-Amino-propyl)-benzolsulfonamid und 2-Oxo-piperidin-1-carbonsäurechlorid, das wiederum aus der Na-Verbindung des 2-Oxo-piperidins und Phosgen zu erhalten ist) werden in 100 ml

Aceton mit 2 g gemahlenem Kaliumcarbonat mehrere Stunden unter Rühren am Rückflußkühler zum Sieden erhitzt. Dann gibt man 0,6 g Cyclohexylisocyanat zu und kocht weitere 6 Std. am Rückfluß. Anschließend dampft man das Aceton unter vermindertem Druck ab, behandelt den Rückstand mit Wasser und Salzsäure, saugt ab und fällt aus verdünntem Ammoniak um. Der N-(4-⟨2-(2-Oxo-piperidin-1-carboxamido)-propyl⟩-benzolsulfonyl)-N'-cyclohexyl-harnstoff wird aus verd. Äthanol umkristallisiert und schmilzt bei 176–177°.

Beispiel 14

N-(4-⟨2-(4-Methyl-2-oxo-piperidin-1-carboxamido)-äthyl⟩-benzolsulfonyl)-N'-cyclohexyl-harnstoff

1,2 g N-(4-⟨2-(4-Methyl-2-oxo-piperidin-1-carboxamido)-äthyl⟩-benzolsulfonyl)-N'-cyclohexyl-thioharnstoff (Schmp. 179–181°, hergestellt aus 4-⟨2-(4-Methyl-2-oxo-piperidin-1-carboxamido)-äthyl⟩-benzolsulfonamid und Cyclohexylsenföl in Aceton in Gegenwart von Pottasche) werden in 75 ml Aceton und 10 ml Wasser gelöst. Zu der Lösung gibt man bei 0° eine Lösung von 0,1 g Natriumnitrit in 5 ml Wasser zu, tropft danach 0,8 ml 5 n Essigsäure ein und rührt 2 Std. bei 0° nach. Anschließend dampft man das Aceton unter vermindertem Druck ab, saugt das Produkt ab und fällt aus verdünntem Ammoniak um. Nach dem Umkristallisieren aus verdünntem Aceton schmilzt der N-(4-⟨2-(4-Methyl-2-oxo-piperidin-1-carboxamido)-äthyl⟩-benzolsulfonyl)-N'-cyclohexyl-harnstoff bei 176–178° und gibt mit dem nach Beispiel 8 hergestellten Produkt keine Depression.

Beispiel 15

N-(4-⟨2-(4-Methyl-2-oxo-piperidin-1-carboxamido)-äthyl⟩-benzolsulfonyl)-N'-cyclohexyl-harnstoff

0,5 g N-(4-⟨2-(4-Methyl-2-oxo-piperidin-1-carboxamido)-äthyl⟩-benzolsulfonyl)-N'-cyclohexyl-isoharnstoffmethyläther (Schmp. 138–140°, hergestellt durch Entschwefelung des N-(4-⟨2-(4-Methyl-2-oxo-piperidin-1-carboxamido)-äthyl⟩-benzolsulfonyl)-N'-cyclohexyl-thioharnstoff mit Quecksilberoxid in Gegenwart von Methanol) werden in 5 ml Dioxan gelöst und mit 2 ml konz. Salzsäure kurz auf dem Dampfbad erhitzt. Anschließend versetzt man das Reaktionsgemisch mit Wasser, saugt ab, fällt aus verdünntem Ammoniak um und kristallisiert aus verd. Aceton um. Der erhaltene N-(4-⟨2-(4-Methyl-2-oxo-piperidin-1-carboxamido)-äthyl⟩-benzolsulfonyl)-N'-cyclohexyl-harnstoff schmilzt bei 176–178° und gibt mit der nach Beispiel 8 erhaltenen Substanz keine Depression.

Beispiel 16

N-(4-⟨2-(4-Methyl-2-oxo-piperidin-1-carboxamido)-äthyl⟩-benzolsulfonyl)-N'-cyclohexyl-harnstoff

0,5 g N-(4-⟨2-(4-Methyl-2-oxo-piperidin-1-carboxamido)-äthyl⟩-benzolsulfonyl)-N'-cyclohexyl-isothioharnstoff-methyläther (Schmp. 167–169°, hergestellt durch Methylierung von N-(4-⟨2-(4-Methyl-2-oxo-piperidin-1-carboxamido)-äthyl⟩-benzolsulfonyl)-N'-cyclohexyl-thioharnstoff mit Methyljodid) werden in 30 ml Tetrahydrofuran mit 2 ml 2n NaOH versetzt und einige Minuten auf dem Dampfbad erhitzt. Anschließend verdünnt man mit Wasser, destilliert das Tetrahydrofuran unter vermindertem Druck ab und säuert mit verdünnter Salzsäure an. Das abgesaugte Produkt wird abgesaugt, aus verdünntem Ammoniak umgefällt und aus verd. Aceton umkristallisiert. Der erhaltene N-(4-⟨2-(4-Methyl-2-oxo-piperidin-1-carboxamido)-äthyl⟩-benzolsulfonyl)-N'-cyclohexyl-harnstoff schmilzt bei 175–177° und gibt mit der nach Beispiel 8 erhaltenen Substanz keine Depression.

Beispiel 17

N-(4-⟨2-(4-Methyl-2-oxo-piperidin-1-carboxamido)-äthyl⟩-benzolsulfonyl)-N'-cyclohexyl-harnstoff

1,6 g N-(4-⟨2-Amino-äthyl⟩-benzolsulfonyl)-N'-cyclohexyl-harnstoff werden mit 0,2 g Natriumhydroxid in 10 ml Wasser und 50 ml Aceton in Lösung gebracht. Zu dieser Lösung tropft man unter Rühren und Eiskühlung eine Lösung von 0,85 g 4-Methyl-2-oxo-piperidin-1-carbonsäurechlorid (hergestellt durch Umsetzung der Natriumverbindung von 4-Methyl-2-oxo-piperidin mit Phosgen) in 20 ml Aceton

und rührt 2 Stunden bei Raumtemperatur nach. Anschließend dampft man das Aceton unter vermindertem Druck ab, säuert an, saugt ab, fällt das Produkt aus verdünntem Ammoniak um und kristallisiert aus verdünntem Aceton um. Der erhaltene N-(4-(2-(4-Methyl-2-oxo-piperidin-1-carboxamido)-äthyl)-benzolsulfonyl)-N'-cyclohexyl-harnstoff schmilzt bei 177—178° und ist mit dem nach Beispiel 8 erhaltenen Produkt identisch.

Beispiel 18

N-(4-(2-(4-Methyl-2-oxo-piperidin-1-carboxamido)-äthyl)-benzolsulfonyl)-
N'-butyl-harnstoff

0,6 g 4-(2-(4-Methyl-2-oxo-piperidin-1-carboxamido)-äthyl)-benzolsulfinsäure (Rohprod. v. Schmp. 113—115°, hergestellt durch Umsetzung von 4-(2-(4-Methyl-2-oxo-piperidin-1-carboxamido)-äthyl)-benzolsulfochlorid mit Natriumsulfit) und 0,3 g N-Hydroxy-N'-butyl-harnstoff werden in 50 ml Dioxan gelöst, zu dieser Lösung tropft man 0,4 g Thionylchlorid in 10 ml Dioxan und erhitzt 2 Std. auf 60°. Anschließend wird das Lösungsmittel unter vermindertem Druck abgedampft, der Rückstand mit Wasser versetzt, die schmierige Substanz abgetrennt, und aus Ammoniak umgefällt. Der erhaltene N-(4-(2-(4-Methyl-2-oxo-piperidin-1-carboxamido)-äthyl)-benzolsulfonyl)-N'-butyl-harnstoff schmilzt bei 155—157° und gibt mit der nach Beispiel 8 erhaltenen Substanz keine Depression.

Beispiel 19

N-(4-(2-(3-Äthyl-4-methyl-2-oxo-3-pyrrolin-1-carboxamido)-äthyl)-benzolsulfonyl)-
N'-cyclohexyl-harnstoff

Entsprechend Beispiel 2 erhält man aus 3-Äthyl-4-methyl-2-pyrrolon und 2-Phenyläthylisocyanat das 3-Äthyl-4-methyl-2-oxo-3-pyrrolin-1-(N-2-phenyläthyl)-carboxamid vom Schmp. 106—108°, daraus mit Chlorsulfonsäure das Sulfochlorid vom Schmp. 172—175°, aus dem Sulfochlorid mit Ammoniak das Sulfonamid vom Schmp. 180—182°, und aus dem Sulfonamid mit Cyclohexylisocyanat den

N-(4-(2-(3-Äthyl-4-methyl-2-oxo-3-pyrrolin-1-carboxamido)-äthyl)-benzolsulfonyl)-
N'-cyclohexylharnstoff
vom Schmp. 185—187° (aus verd. Aceton).

In analoger Weise erhält man aus dem Sulfonamid den

N-(4-(2-(3-Äthyl-4-methyl-2-oxo-3-pyrrolin-1-carboxamido)-äthyl)-benzolsulfonyl)-
N'-4-methyl-cyclohexylharnstoff
vom Schmp. 168—170° (aus verd. Aceton),
N-(4-(2-(3-Äthyl-4-methyl-2-oxo-3-pyrrolin-1-carboxamido)-äthyl)-benzolsulfonyl)-
N'-butylharnstoff
vom Schmp. 151—153° (aus Methanol).

Beispiel 20

N-(4-((2-(3-Butyl-4-methyl-2-oxo-3-pyrrolin-1-carboxamido)-äthyl))-benzolsulfonyl)-
N'-cyclohexyl-harnstoff

Entsprechend Beispiel 2 erhält man aus 3-Butyl-4-methyl-2-pyrrolon (Schmp. 95—97°, hergestellt analog A. 598, 198 [1956]) und 2-Phenyläthylisocyanat das 3-Butyl-4-methyl-2-oxo-3-pyrrolin-1-(N-2-phenyläthyl)-carboxamid vom Schmp. 90—91°, daraus mit Chlorsulfonsäure das Sulfochlorid, aus dem rohen Sulfochlorid mit Ammoniak das Sulfonamid vom Schmp. 132—134°, und aus dem Sulfonamid mit Cyclohexylisocyanat den

N-(4-(2-(3-Butyl-4-methyl-2-oxo-3-pyrrolin-1-carboxamido)-äthyl)-benzolsulfonyl)-
N'-cyclohexylharnstoff
vom Schmp. 173—175° (aus verd. Methanol).

In analoger Weise erhält man aus dem Sulfonamid den

N-(4-(2-(3-Butyl-4-methyl-2-oxo-3-pyrrolin-1-carboxamido)-äthyl)-benzolsulfonyl)-
N'-4-methyl-cyclohexylharnstoff

vom Schmp. 178–180° (aus verd. Methanol),
N-(4-(2-(3-Butyl-4-methyl-2-oxo-3-pyrrolin-1-carboxamido)-äthyl)-benzolsulfonyl)-N'-butylharnstoff
vom Schmp. 127–129° (aus verd. Methanol).

In analoger Weise erhält man aus dem im Beispiel 11 erwähnten Sulfonamid den

N-(4-(2-(3,4-Dimethyl-2-oxo-3-pyrrolin-1-carboxamido)-äthyl)-benzolsulfonyl)-N'-4-äthyl-cyclohexylharnstoff
vom Schmp. 203–205° C (aus Methanol/Dioxan).

**Patentansprüche für die Vertragsstaaten: BE, CH, DE, FR, GB, IT, LI, LU, NL, SE**

1. Sulfonylharnstoffe der Formel

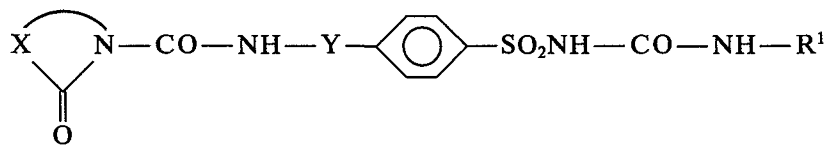

in welcher bedeuten:

X a) einen Alkylenrest mit 3 bis 6 C-Atomen, der gegebenenfalls mit bis zu 3 Alkylgruppen mit je 1 bis 4 C-Atomen oder mit einem Phenylrest substituiert ist,
b) einen Alkenylenrest mit 3 bis 6 C-Atomen, der gegebenenfalls mit bis zu 3 Alkylgruppen mit je 1 bis 4 C-Atomen oder mit einem Phenylrest substituiert ist, oder

die Gruppe

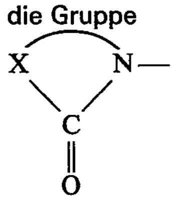

ist ein bicyclisches System der Formel

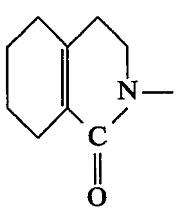

oder

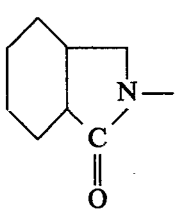

Y Alkylen mit 2 bis 3 C-Atomen
$R^1$ Alkyl mit 4 bis 6 C-Atomen, Cycloalkyl, Alkylcycloalkyl, Dialkylcycloalkyl, Cycloalkylalkyl, Cycloalkenyl, Alkylcycloalkenyl mit jeweils 4 bis 9 C-Atomen, Methylcyclopentylmethyl, Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Notricyclyl, Adamantyl oder Benzyl, und deren physiologisch verträgliche Salze.

2. Verfahren zur Herstellung von Sulfonylharnstoffen gemäß Anspruch 1, dadurch gekennzeichnet,

daß man

a) mit der Gruppe

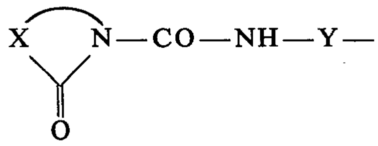

in 4-Stellung substituierte Benzolsulfonyl-isocyanate, -carbaminsäureester, -thiocarbaminsäureester, -harnstoffe, -semicarbazide oder -semicarbazone mit einem Amin $R^1$—$NH_2$ oder dessen Salzen umsetzt oder Sulfonamide der Formel .

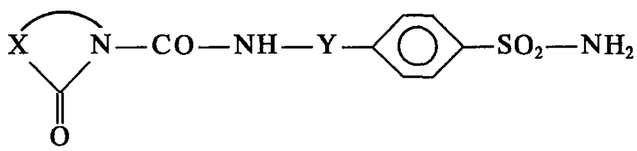

oder deren Salze mit $R^1$-substituierten Isocyanaten, Carbaminsäureestern, Thiolcarbaminsäureestern, Carbaminsäurehalogeniden oder Harnstoffen umsetzt,

b) mit der Gruppe

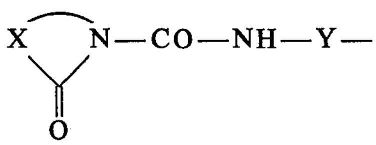

substituierte Benzolsulfonyl-isoharnstoffäther, -isothioharnstoffäther, -parabansäuren oder -halogenameisensäureamidine oder mit der Gruppe

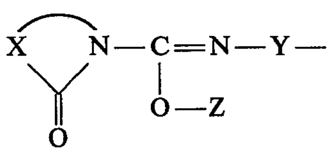

worin Z Alkyl mit 1 bis 2 Kohlenstoffatomen bedeutet, substituierte Benzolsulfonylharnstoffe spaltet,

c) in

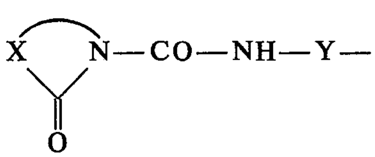

substituierten Benzolsulfonylthioharnstoffen das Schwefelatom durch Sauerstoff ersetzt,

d) entsprechende Benzolsulfinyl- oder -sulfenyl-harnstoffe oxydiert,

e) in Benzolsulfonylharnstoffe der Formel

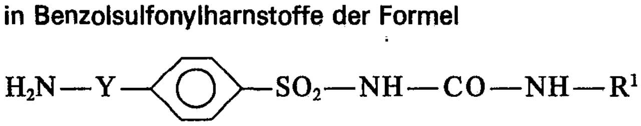

den Rest

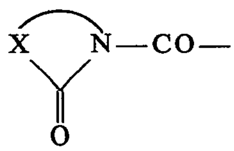

einführt,

f) entsprechend substituierte Benzolsulfonylhalogenide mit $R^1$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit $N-R^1-N'$-hydroxy-harnstoff umsetzt und die Reaktionsprodukte gegebenenfalls zur Salzbildung mit alkalischen Mitteln behandelt.

3. Arzneimittel enthaltend einen Sulfonylharnstoff gemäß Anspruch 1 oder eines seiner Salze.

4. Sulfonylharnstoff gemäß Anspruch 1 oder eines seiner Salze zur Verwendung bei der Behandlung von Diabetes.

**Patentanspruch für den Vertragsstaat: AT**

1. Verfahren zur Herstellung von Sulfonylharnstoffen der Formel

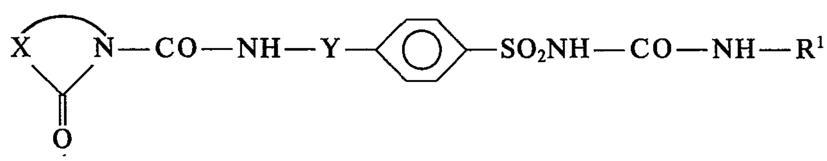

in welcher bedeuten:

X a) einen Alkylenrest mit 3 bis 6 C-Atomen, der gegebenenfalls mit bis zu 3 Alkylgruppen mit je 1 bis 4 C-Atomen oder mit einem Phenylrest substituiert ist,

b) einen Alkenylenrest mit 3 bis 6 C-Atomen, der gegebenenfalls mit bis zu 3 Alkylgruppen mit je 1 bis 4 C-Atomen oder mit einem Phenylrest substituiert ist, oder

die Gruppe

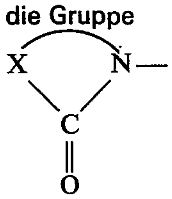

ist ein bicyclisches System der Formel

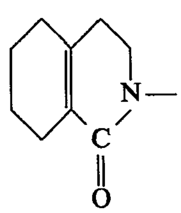

oder

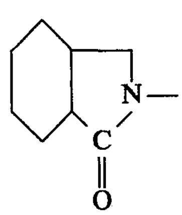

Y Alkylen mit 2 bis 3 C-Atomen

$R^1$ Alkyl mit 4 bis 6 C-Atomen, Cycloalkyl, Alkylcycloalkyl, Dialkylcycloalkyl, Cycloalkylalkyl, Cycloalkenyl, Alkylcycloalkenyl mit jeweils 4 bis 9 C-Atomen, Methylcyclopentylmethyl, Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl, und von deren physiologisch verträglichen Salzen,

dadurch gekennzeichnet, daß man

a) mit der Gruppe

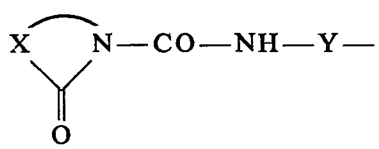

in 4-Stellung substituierte Benzolsulfonyl-isocyanate, -carbaminsäureester, -thiocarbaminsäureester, -harnstoffe, -semicarbazide oder -semicarbazone mit einem Amin $R^1—NH_2$ oder dessen Salzen umsetzt oder Sulfonamide der Formel

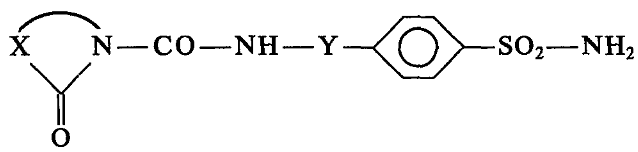

oder deren Salze mit $R^1$-substituierten Isocyanaten, Carbaminsäureestern, Thiolcarbaminsäureestern, Carbaminsäurehalogeniden oder Harnstoffen umsetzt,

b) mit der Gruppe

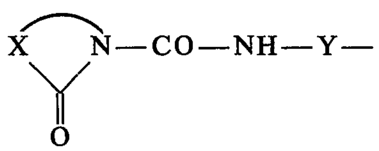

substituierte Benzolsulfonyl-isoharnstoffäther, -isothioharnstoffäther, -parabansäuren oder -halogenameisensäureamidine oder mit der Gruppe

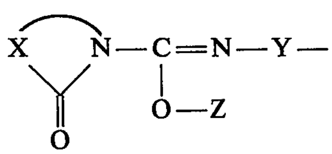

worin Z Alkyl mit 1 bis 2 Kohlenstoffatomen bedeutet, substituierte Benzolsulfonylharnstoffe spaltet,

c) in

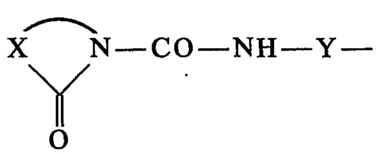

substituierten Benzolsulfonylthioharnstoffen das Schwefelatom durch Sauerstoff ersetzt,

d) entsprechende Benzolsulfinyl- oder -sulfenyl-harnstoffe oxydiert,

e) in Benzolsulfonylharnstoffe der Formel

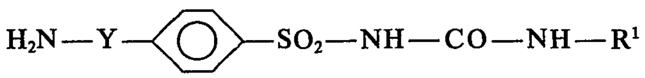

den Rest

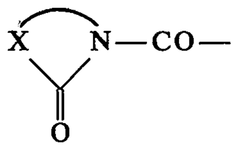

einführt,

f) entsprechend substituierte Benzolsulfonylhalogenide mit $R^1$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit $N-R^1-N'$-hydroxy-harnstoff umsetzt und die Reaktionsprodukte gegebenenfalls zur Salzbildung mit alkalischen Mitteln behandelt.

**Claims for the Contracting states: BE, CH, DE, FR, GB, IT, LI, LU, NL, SE**

1. Sulfonyl ureas of the formula

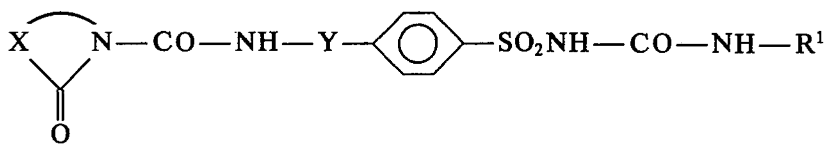

in which

X is
a) an alkylene radical having from 3 to 6 carbon atoms, which is optionally substituted by up to 3 alkyl groups each having from 1 to 4 carbon atoms, or by a phenyl radical,
b) an alkenylene radical having from 3 to 6 carbon atoms, which is optionally substituted by up to 3 alkyl groups each having from 1 to 4 carbon atoms or by a phenyl radical, or

the group

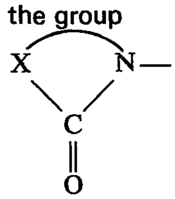

is a bicyclic system of the formula

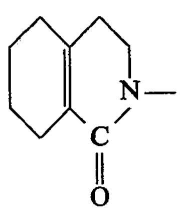

or

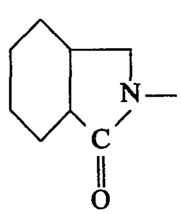

Y is alkylene having 2 or 3 carbon atoms;
$R^1$ is alkyl having from 4 to 6 carbon atoms, cycloalkyl, alkylcycloalkyl, dialkylcycloalkyl,

cycloalkylalkyl, cycloalkenyl or alkylcycloalkenyl with in each case 4 to 9 C-atoms, methylcyclopentylmethyl, cyclohexenylmethyl, chlorocyclohexyl, methoxycyclohexyl, bicycloheptyl, bicycloheptenyl, bicycloheptylmethyl, bicycloheptenylmethyl, bicyclooctyl, nortricyclyl, adamantyl or benzyl;
and the physiologically acceptable salts thereof.

2. Processes for the manufacture of sulfonyl ureas as claimed in Claim 1, which comprises

a) reacting benzenesulfonyl-isocyanates, -carbamic acid esters, -thiolcarbamic acid esters, -ureas, -semicarbazides or -semicarbazones substituted by the group

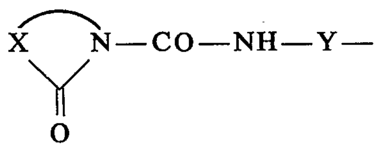

in 4-position, with an amine $R^1-NH_2$ or salts thereof, or reacting sulfonamides of the formula

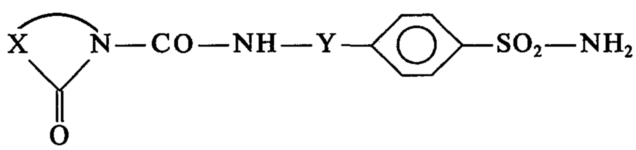

or salts thereof with $R^1$-substituted isocyanates, carbamic acid esters, thiolcarbamic acid esters, carbamic acid halides or ureas,

b) splitting benzenesulfonylisourea ethers, -isothiourea ethers, -parabanic acids or -halogenoformic acid amidines, each substituted by the

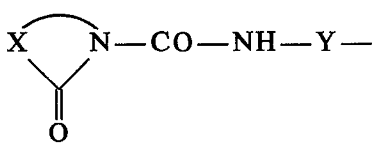 group

or benzenesulfonyl ureas substituted by the

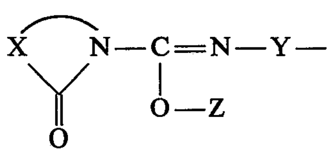

group, where Z is alkyl having 1 or 2 carbon atoms;

c) replacing the sulfur atom in benzensulfonylthioureas substituted by

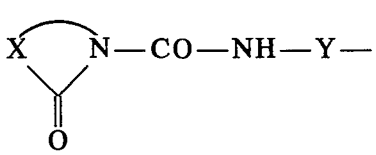

by oxygen,

d) oxidizing corresponding benzene-sulfinyl- or -sulfenylureas,

e) introducing the radical

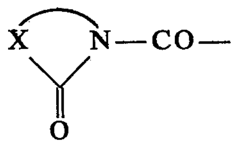

into benzenesulfonylureas of the formula

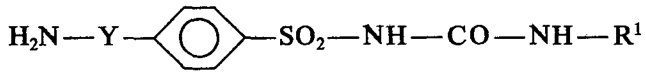

f) reacting correspondingly substituted benzenesulfonyl halides with $R^1$-substituted ureas or alkali metal salts thereof, or reacting correspondingly substituted benzenesulfinic acid halides or, in the presence of acidic condensing agents, also correspondingly substituted sulfinic acids or alkali metal salts thereof with $N-R^1-N'$-hydroxyurea, and, if appropriate, treating the reaction products with alkaline agents in order to form salts.

3. Medicaments containing a sulfonyl urea as claimed in Claim 1 or one of its salts.
4. Sulfonyl urea as claimed in Claim 1 or one of its salts for use in the treatment of diabetes.

**Claim for the Contracting state: AT**

Processes for the manufacture of sulfonyl ureas of the formula

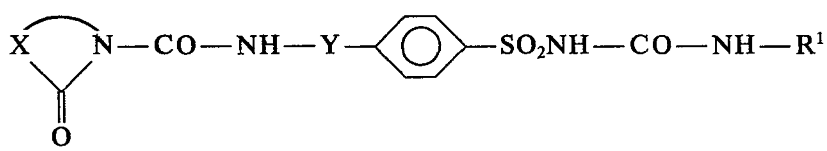

in which

X is
a) an alkylene radical having from 3 to 6 carbon atoms, which is optionally substituted by up to 3 alkyl groups each having from 1 to 4 carbon atoms, or by a phenyl radical,
b) an alkenylene radical having from 3 to 6 carbon atoms, which is optionally substituted by up to 3 alkyl groups each having from 1 to 4 carbon atoms or by a phenyl radical, or

the group

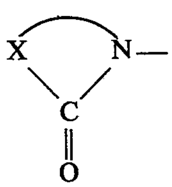

is a bicyclic system of the formula

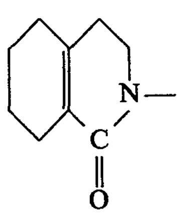

or

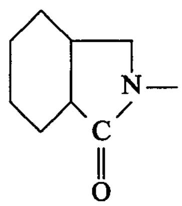

Y is alkylene having 2 or 3 carbon atoms;

$R^1$ is alkyl having from 4 to 6 carbon atoms, cycloalkyl, alkylcycloalkyl, dialkylcycloalkyl, cycloalkylalkyl, cycloalkenyl or alkylcycloalkenyl with in each case 4 to 9 C-atoms, methylcyclopentylmethyl, cyclohexenylmethyl, chlorocyclohexyl, methoxycyclohexyl, bicycloheptyl, bicycloheptenyl, bicycloheptylmethyl, bicycloheptenylmethyl, bicyclooctyl, nortricyclyl, adamantyl or benzyl;
and of the physiologically acceptable salts thereof,

which comprises

a) reacting benzenesulfonyl-isocyanates, -carbamic acid esters, -thiolcarbamic acid esters, -ureas, -semicarbazides or -semicarbazones substituted by the group

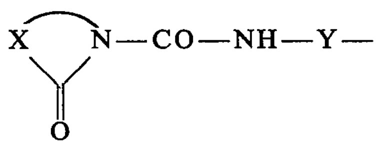

in 4-position, with an amine $R^1—NH_2$ or salts thereof, or reacting sulfonamides of the formula

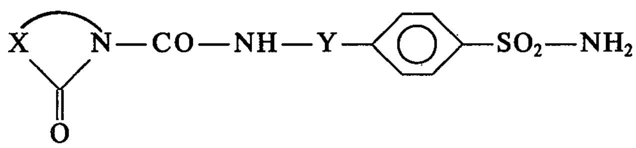

or salts thereof with $R^1$-substituted isocyanates, carbamic acid esters, thiolcarbamic acid esters, carbamic acid halides or ureas,

b) splitting benzenesulfonylisourea ethers, -isothiourea ethers, -parabanic acids or -halogenoformic acid amidines, each substituted by the

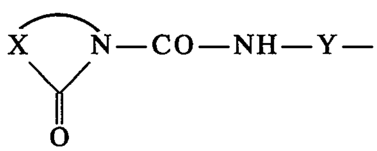 group

or benzenesulfonyl ureas substituted by the

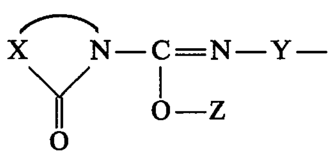

group, where Z is alkyl having 1 or 2 carbon atoms;

c) replacing the sulfur atom in benzensulfonylthioureas substituted by

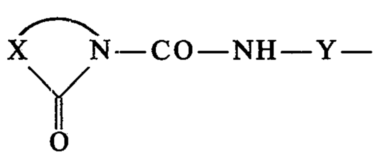

by oxygen,

d) oxidizing corresponding benzene-sulfinyl- or -sulfenylureas,

e) introducing the radical

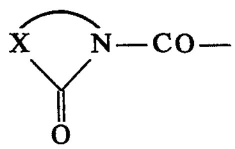

into benzenesulfonylureas of the formula

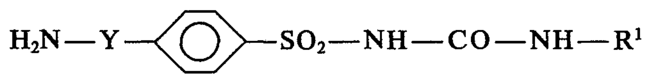

f) reacting correspondingly substituted benzenesulfonyl halides with $R^1$-substituted ureas or alkali metal salts thereof, or reacting correspondingly substituted benzenesulfinic acid halides or, in the presence of acidic condensing agents, also correspondingly substituted sulfinic acids or alkali metal salts thereof with $N-R^1-N'$-hydroxyurea, and, if appropriate, treating the reaction products with alkaline agents in order to form salts.

**Revendications pour les Etats contractants: BE, CH, DE, FR, GB, IT, LI, LU, NL, SE**

1. Sulfonylurées, répondant à la formule

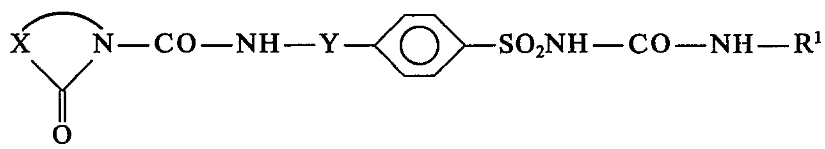

dans laquelle:

X représente

a) un groupe alcoylène ayant de 3 à 6 atomes de carbone, qui est éventuellement substitué avec au maximum 3 groupes alcoyle ayant de 1 à 4 atomes de carbone ou avec un groupe phényle,

b) un groupe alcénylène ayant de 3 à 6 atomes de carbone, qui est éventuellement substitué avec au maximum 3 groupes alcoyle ayant de 1 à 4 atomes de carbone ou avec un groupe phényle, ou

le groupe

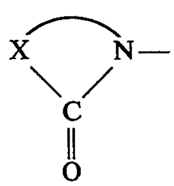

est un système bicyclique de formule

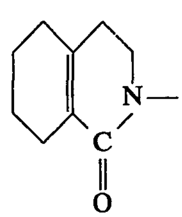

ou

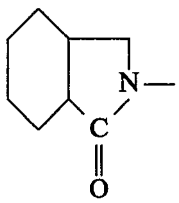

Y représente un groupe alcoylène ayant de 2 à 3 atomes de carbone,

$R^1$ représente un groupe alcoyle ayant de 4 à 6 atomes de carbone, un groupe cycloalcoyle, alcoylcycloalcoyle, dialcoylcycloalcoyle, cycloalcoylalcoyle, cycloalcényle, alcoylcycloalcényle ayant chacun de 4 à 9 atomes de carbone, un groupe méthylcyclopentylméthyle, cyclohexénylméthyle, chlorocyclohexyle, méthoxycyclohexyle, bicycloheptyle, bicycloheptényle, bicycloheptylméthyle, bicycloheptènylméthyle, bicyclooctyle, nortricyclyle, adamantyle ou benzyle,

et leurs sels physiologiquement acceptables.

2. Procédé pour la préparation de sulfonylurées selon la revendication 1, caractérisé en ce que:

a) on fait réagir des benzène sulfonyl-isocyanates, des esters de l'acide benzènesulfonyl-carbamique des esters de l'acide benzène sulfonyl-thiocarbamique, des benzène sulfonyl-urées, des benzène sulfonyl-semicarbazides ou des benzène sulfonyl-semicarbazones, tous substitués en position 4, avec le groupe

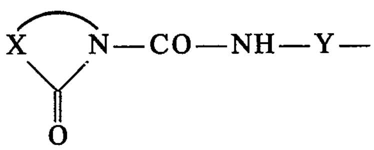

avec une amine $R^1—NH_2$ ou un de ses sels, ou on fait réagir des sulfamides de formule

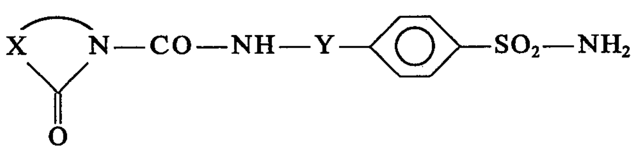

ou leurs sels avec des isocyanates, des esters de l'acide carbamique, des esters de l'acide thiocarbamique, des halogénures de l'acide carbamique ou des urées, tous $R^1$-substitués,

b) on sépare des benzène sulfonyl-isourée éthers, des benzène sulfonyl-isothiourée éthers, des acides benzène sulfonyl-parabaniques ou des amidines d'acides benzène sulfonyl-halogénoformiques substitués avec le groupe

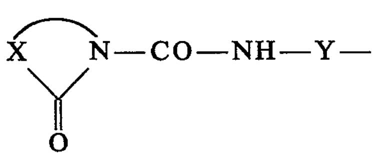

ou on sépare des benzène sulfonylurées substituées avec le groupe

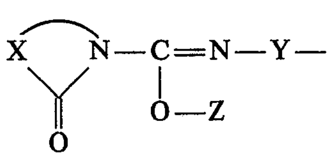

dans lequel Z représente un groupe alcoyle ayant de 1 à 2 atomes de carbone,

c) dans les benzène sulfonylthiourées substituées avec le groupe

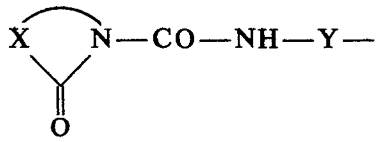

on remplace l'atome de soufre par un atome d'oxygène,

d) on oxyde des benzène sulfinyl- ou sulfénylurées correspondantes,

e) dans les benzène sulfonylurées de formule

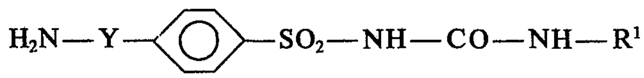

on introduit le groupe

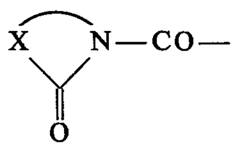

f) on fait réagir des halogénures de benzène sulfonyle substitués correspondants avec des urées $R^1$-substituées ou leurs sels alcalins ou on fait réagir des halogénures d'acides benzène sulfiniques substitués correspondants, ou en présence d'agents de condensation acides, également des acides sulfiniques substitués correspondants ou leurs sels alcalins, avec une $N-R^1-N'$-hydroxyurée et éventuellement on traite les produits de réaction avec des agents alcalins pour former des sels.

3. Médicament contenant une sulfonylurée selon la revendication 1 ou un de ses sels.
4. Sulfonylurée selon la revendication 1 ou un de ses sels pour utilisation dans le traitement du diabète.

**Revendication pour l'Etat contractant: AT**

Procédé pour la préparation de sulfonylurées répondant à la formule

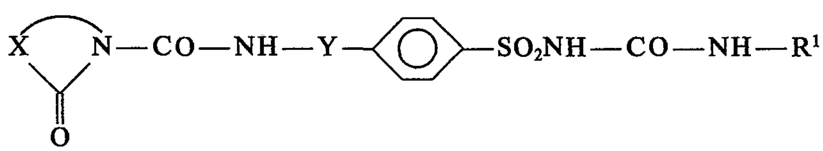

dans laquelle

X représente:
a) un groupe alcoylène ayant de 3 à 6 atomes de carbone, qui est éventuellement substitué avec au maximum 3 groupes alcoyle ayant de 1 à 4 atomes de carbone ou avec un groupe phényle,
b) un groupe alcénylène ayant de 3 à 6 atomes de carbone, qui est éventuellement substitué avec au maximum 3 groupes alcoyle ayant de 1 à 4 atomes de carbone ou avec un groupe phényle, ou

le groupe

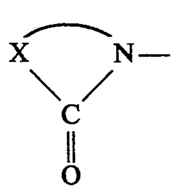

est un système bicyclique de formule

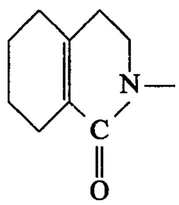

ou

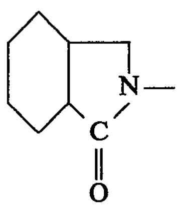

Y représente un groupe alcoylène ayant de 2 à 3 atomes de carbone,

$R^1$ représente un groupe alcoyle ayant de 4 à 6 atomes de carbone, un groupe cycloalcoyle, alcoylcycloalcoyle, dialcoylcycloalcoyle, cycloalcoylalcoyle, cycloalcényle, alcoylcycloalcényle ayant chacun de 4 à 9 atomes de carbone, un groupe méthylcyclopentylméthyle, cyclohexénylméthyle, chlorocyclohexyle, méthoxycyclohexyle, bicycloheptyle, bicycloheptényle, bicycloheptylméthyle, bicyclohepténylméthyle, bicyclooctyle, nortricyclyle, adamantyle ou benzyle,

et de leurs sels physiologiquement acceptables,

caractérisé en ce que:

a) on fait réagir des benzène sulfonylisocyanates, des esters de l'acide benzène sulfonyl-carbamique, des esters de l'acide benzène sulfonyl-thiocarbamique, des benzène sulfonylurées, des benzène sulfonyl-semicarbazides ou des benzène sulfonyl-semicarbazones, tous substitués en position 4 avec le groupe

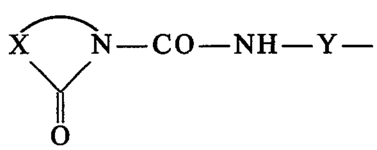

avec une amine $R^1-NH_2$ ou ses sels ou on fait réagir des sulfamides de formule

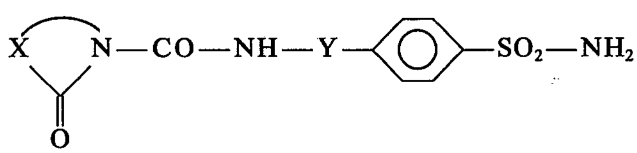

ou leurs sels avec des isocyanates, des esters de l'acide carbamique, des esters de l'acide thiocarbamique, des halogénures de l'acide carbamique ou des urées, tous $R^1$-substitués,

b) on sépare des benzène sulfonylisourée éthers, des benzène sulfonyl-isothiourée éthers, des acides benzène sulfonyl-parabaniques ou des amidines d'acides benzène sulfonyl-halogénoformiques substitués avec le groupe

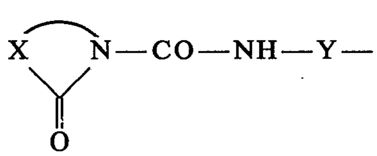

ou on sépare des benzène sulfonylurées substituées avec le groupe

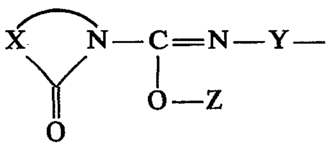

dans lequel Z représente un groupe alcoyle ayant de 1 à 2 atomes de carbone,

c) dans les benzène sulfonylthiourées substituées avec le groupe

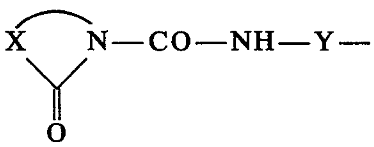

on remplace l'atome de soufre par un atome d'oxygène,

d) on oxyde des benzène sulfinyl- ou sulfénylurées correspondantes,

e) dans les benzène sulfonylurées de formule

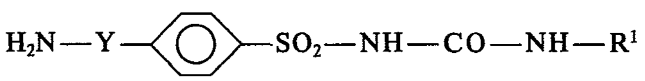

on introduit le groupe

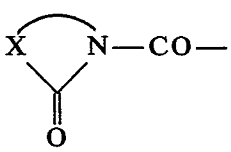

f) on fait réagir des halogénures de benzène sulfonyle substitués correspondants avec des urées $R^1$-substituées ou leurs sels alcalins ou on fait réagir des halogénures d'acides benzène sulfiniques substitués correspondants ou, en présence d'agents de condensation acides, également des acides sulfiniques substitués correspondants ou leurs sels alcalins, avec la $N-R^1-N'$-hydroxy-urée et éventuellement on traite les produits de réaction avec des agents alcalins pour former des sels.